(19) Europäisches Patentamt
European Patent Office
Office européen des brevets

(11) Veröffentlichungsnummer: **0 031 303**
**A2**

(12) # EUROPÄISCHE PATENTANMELDUNG

(21) Anmeldenummer: 80810395.6

(22) Anmeldetag: 15.12.80

(51) Int. Cl.³: C 07 C 103/52
A 61 K 37/02

(30) Priorität: 21.12.79 CH 11409 79

(43) Veröffentlichungstag der Anmeldung: 01.07.81 Patentblatt 81/26

(84) Benannte Vertragsstaaten: AT BE CH DE FR GB IT LI LU NL SE

(71) Anmelder: CIBA-GEIGY AG
Patentabteilung Postfach
CH-4002 Basel(CH)

(72) Erfinder: Sieber, Peter
Bachmattweg 10
CH-4153 Reinach(CH)

(72) Erfinder: Kamber, Bruno, Dr.
Sonnenweg 9
CH-4144 Arlesheim(CH)

(72) Erfinder: Rink, Hans
Rebenstrasse 10
CH-4125 Riehen(CH)

(54) **Cyclooctapeptide und pharmazeutische Präparate davon, sowie Verfahren zur Herstellung derselben und ihre Anwendung.**

(57) Cyclische Peptide der Formel

$$\overline{\text{Phe-Phe-trp-Lys}(Ac_A)\text{-Thr-Phe-A-B}} \quad (I)$$

6 7 8 9 10 11 12 13

worin trp für L-Trp, D-Trp, oder einen analogen Rest, der im Indolkern ein Halogen trägt, $Ac_A$ für Wasserstoff oder einen an der ε-Aminogruppe befindlichen Acylrest Ac einer Carbonsäure, A für L-Phe, D-Phe, L-Phg oder D-Phg, oder einen entsprechenden Rest mit substituiertem oder gesättigtem Phenylring, und B für den Rest einer γ- oder δ-Aminoniederalkancarbonsäure, welche einen gegebenenfalls substituierten cyclischen Hydrocarbylrest Ar tragen kann, steht, sowie pharmakolologisch verträgliche Salze und therapeutisch verwendbare Komplexe davon sind als Antidiabeticum in analoger Weise wie Somatostatin anwendbar. Sie sind durch konventionelle Verfahren der Peptidchemie, insbesondere durch die Cyclisierung entsprechender linearer Peptide, erhältlich.

EP 0 031 303 A2

Croydon Printing Company Ltd.

CIBA-GEIGY AG 4-12661/=
Basel (Schweiz)

Cyclooctapeptide und pharmazeutische Präparate davon, sowie Verfahren zur Herstellung derselben und ihre Anwendung.

Die Erfindung betrifft neue Cyclopeptide vom Somatostatin-Typ und ihre Herstellungsverfahren, sowie pharmazeutische Präparate enthaltend diese Verbindungen und Verwendung dieser Verbindungen bzw. Präparate zu therapeutischen Zwecken.

Die Erfindung betrifft insbesondere Cyclopeptide, welche die wesentlichsten Merkmale des Somatostatins, wie die Teilsequenz der Aminosäuren 6-11 oder eine äquivalente Sequenz, enthalten, dabei aber schwefelfrei sind. Die erfindungsgemässen Somatostatin-analogen Cyclopeptide umfassen cyclische Octapeptide der Formel

$$\underbrace{\overset{\ }{\text{Phe}}}_{6} - \underbrace{\text{Phe}}_{7} - \underbrace{\text{trp}}_{8} - \underbrace{\text{Lys}(\text{Ac}_A)}_{9} - \underbrace{\text{Thr}}_{10} - \underbrace{\text{Phe}}_{11} - \underbrace{\text{A}}_{12} - \underbrace{\text{B}}_{13} \quad \text{(cyclisch)} \qquad (I)$$

worin trp für L-Trp, D-Trp, oder einen analogen Rest, der im Indolkern ein Halogen trägt, $Ac_A$ für einen an der ε-Aminogruppe befindlichen Acylrest Ac einer Carbonsäure oder vorzugsweise Wasserstoff, A für L-Phe, D-Phe, L-Phg oder D-Phg (wobei Phg einen C-Phenylglycylrest bedeutet), oder einen entsprechenden Rest mit substituiertem oder gesättigtem Phenylring und B für den Rest einer γ- oder δ-Aminoniederalkancarbonsäure, welche einen gegebenenfalls substituierten cyclischen Hydrocarbylrest Ar an einem ihrer C-Atome tragen kann, steht, sowie Salze und Komplexe davon.

Somatostatin, ein cyclisches Tetradecapeptid der Formel

$$\text{H-Ala-Gly-}\underset{3}{\text{Cys}}\text{-Lys-Asn-Phe-Phe-Trp-Lys-Thr-Phe-Thr-Ser-}\underset{14}{\text{Cys}}\text{-OH}$$

(Ala 1, Gly 2, Cys 3, Lys 4, Asn 5, Phe 6, Phe 7, Trp 8, Lys 9, Thr 10, Phe 11, Thr 12, Ser 13, Cys 14; Brücke zwischen Cys 3 und Cys 14)

[Science 179,77 (1973)] hemmt bekanntlich die Hypophysengesteuerte Sekretion des Wachstumshormons (Somatotropin). Ausserdem hemmt es die sekretorische Aktivität des endocrinen Pancreas, wie die Sekretion von Insulin und Glucagon. Diese wertvollen Eigenschaften können beim Somatostatin selber nicht zur vollen praktischen Anwendung gelangen, da diese Verbindung eine zu kurze Wirkungsdauer aufweist. Zudem ist es oft bevorzugt, wenn der Wirkstoff seine hemmende Wirkung hauptsächlich auf eine der beiden Drüsen ausübt, wogegen die andere Drüse möglichst wenig beeinflusst werden sollte. (In den meisten Fällen ist die Hemmung der hypophysären Sekretion, d.h. die der Wachstumshormon-Ausschüttung, weniger gewünscht.) Deshalb wird angestrebt, durch Modifizierung der Grundsequenz, insbesondere durch Auslassen einzelner ursprünglicher Aminosäuren und/oder deren Austausch gegen andere, oft auch "unnatürliche" Aminosäuren, eine Dissociation der Hemmwirkungen sowie eine möglichst lange Wirkungsdauer zu erlangen. So wurde z.B. gefunden, dass besonders vorteilhafte physiologische Eigenschaften dieser Art bei Cyclopeptiden vom Typ

$$\overline{\text{Asn-Phe-Phe-[D-trp]-Lys-Thr-Phe-[Gaba]}_p} \qquad (M)$$

5 6 7 8 9 10 11 12

worin Gaba den Rest der γ-Aminobuttersäure und p die Zahl 0, 1 oder 2 darstellt, und weiteren nahe verwandten Verbindungen auftreten, vgl. unsere Europäische Patentanmeldung Nr. 78 100 994.9, veröffentlicht unter Nr. 0 001 295. - Als ein typisches Merkmal dieser Verbindungen kommt das Vorhandensein einer geraden Kohlenstoffkette vor, welche die ursprüngliche Gruppierung $-CH_2-S-S-CH_2-$ im Cystinrest von Somatostatin simulieren soll.

Ueberraschenderweise wurde nun festgestellt, dass durch eine ganz ungewöhnliche Modifizierung dieses einfachen, jeweils unsubstituierten Kettenabschnittes, durch Substitution mit einem cyclischen Hydrocarbylrest, die gewünschte Aktivität nicht nur erhalten bleibt, sondern noch weiter gesteigert

BAD ORIGINAL

wird. Beispielsweise wurde bei der Bestimmung der Insulin- und Glucagon-Ausschüttung am isolierten, perfundierten, mit Arginin stimulierten Ratten-Pankreas [Methode gemäss B. Petrack, A.J. Czernik, W. Itterly, J. Ansell und H. Chertock: Biochem. and Biophys. Res. Commun. 73, 934-939 (1976)] gefunden, dass das [D-$Trp^8$, $Phe^{12}$, $Gaba^{13}$]-cyclo-somatostatin(6-13)-octapeptid eine 1-2fache Hemmwirkung auf die Insulin- und Glucagon-Sekretion, verglichen mit Somatostatin, ausübt.

In den eingangs definierten Verbindungen der Formel I bedeutet trp vorzugsweise D-Trp oder aber einen L- oder D-Trp-Rest, der im Indolkern, insbesondere in der 5-Stellung, ein Halogenatom, wie Chlor oder insbesondere Fluor, trägt, z.B. insbesondere [D-(5F)Trp], der sich von D-5-Fluortryptophan ableitet.

In den eingangs definierten Verbindungen der Formel I ist unter den Bedeutungen von A besonders der Rest L-Phe, weiter auch D-Phe hervorzuheben. Ein Rest, der Phe oder Phg entspricht und im Phenylring gesättigt ist, ist ein β-Cyclohexylalanyl- (Cha) bzw. C-Cyclohexylglycyl-Rest, diese können in der L- oder D-Konfiguration vorliegen. Ein Rest, der dem Rest Phe oder Phg entspricht, und im Phenylring substituiert ist, ist ein solcher, der einen oder zwei gleiche oder verschiedene der weiter unten angegebenen Substituenten eines aromatischen Rings trägt, z.B. L- oder D-Tyr.

Eine unter dem Symbol B in der Formel I angegebene γ- oder δ-Aminoniederalkancarbonsäure ist eine solche mit 4-8, insbesondere mit 4- oder 5, Kohlenstoffatomen, vorzugsweise eine solche mit gerader Kohlenstoffkette, insbesondere die δ-Aminovaleriansäure und vor allem die γ-Aminobuttersäure (Gaba). Falls eine solche Säure einen Hydrocarbylrest Ar trägt, befindet sich dieser vorzugsweise in der β-Stellung. Ein derartiger besonders bevorzugter Rest ist derjenige der β-Ar-γ-aminobuttersäure [Gaba(Ar)]. Ganz bevorzugt sind jedoch Reste der gesagten Aminoniederalkansäuren, die unsubstituiert sind, wie vor allem Gaba.

BAD ORIGINAL

Der cyclische Hydrocarbylrest Ar ist insbesondere ein mono- oder bicyclischer Cycloalkylrest oder ein entsprechender, mindestens einen aromatischen Ring enthaltender Arylrest mit höchstens 12 Ringkohlenstoffatomen. Unter den Cycloalkylresten sind bevorzugt solche mit 3- bis 8-, und vor allem 5- und/oder 6-gliedrigen Ringen, wie beispielsweise Cyclopropyl, Cyclobutyl, Cycloheptyl, Cyclooctyl und ganz besonders Cyclopentyl und Cyclohexyl. Ein Arylrest ist ein Naphthyl, wie 1- oder 2-Naphthyl, ein entsprechendes teilweise hydriertes Naphthyl, wie insbesondere 1-, 2-, 5- oder 6-(1,2,3,4-Tetrahydronaphthyl) oder ein Phenyl. Diese cyclischen Hydrocarbylreste können einen oder mehrere Alkylreste mit höchstens 4 Kohlenstoffatomen, z.B. Methyl, Aethyl, Propyl, Isopropyl oder ein Butyl, und/oder weitere cyclische, insbesondere monocyclische Hydrocarbylreste, wie die oben definierten, tragen, wobei die Gesamtzahl der Kohlenstoffatome höchstens 18 Kohlenstoffatome beträgt. Als solche cyclische Hydrocarbylreste sind beispielsweise 4,4-Dimethylcyclohexyl, ein Tolyl, wie 2-, 3- oder 4-Tolyl und ein Biphenylyl, z.B. 4-Biphenylyl, zu nennen.

In ihrem aromatischen Teil können die cyclischen Hydrocarbylreste durch ein, zwei oder mehrere, gleiche oder verschiedene Substituenten substituiert sein, wie Halogen, z.B. Chlor, Brom, Jod und insbesondere Fluor, Niederalkoxy, insbesondere ein solches, das sich von einem der obengenannten Niederalkylreste mit höchstens 4 Kohlenstoffatomen ableitet, darunter vor allem Methoxy, ferner auch Nitro und Amino, insbesondere primäres Amino, Diniederalkylamino und Acylamino-, wie Niederalkanoylamino, z.B. Acetamino. Besonders bevorzugt sind durch die genannten Substituenten substituierte Phenylreste. (In gleicher Weise können auch die Phenylringe von Phe und Phg, wie sie unter den Bedeutungen von A erwähnt sind, einen oder zwei gleiche oder verschiedene der genannten Substituenten, insbesondere einen in der 4-Stellung, tragen.)

BAD ORIGINAL

Besonders bevorzugte Reste der Formel Gaba(Ar) leiten sich von den folgenden Buttersäuren: 4-Amino-3-phenyl-, 4-Amino-3-cyclohexyl-, 4-Amino-3-(2-naphthyl)- und vor allem 4-Amino-3-(1-naphthyl)-buttersäure.

Als Resultat der Substitution mit dem Rest Ar entsteht am entsprechenden Kohlenstoffatom der Säurekette ein Asymmetriezentrum, welches das Vorliegen von jeweils zwei diastereomeren Formen des erfindungsgemässen Cyclopeptids zu Folge hat, die gewünschtenfalls voneinander getrennt, oder aber gemeinsam als Diastereomerengemisch für dieselben Verwendungszwecke eingesetzt werden können.

Falls in der Formel I das Symbol $Ac_A$ für Wasserstoff steht, bedeutet es, dass die endständige Aminogruppe des Lys-Rests frei ist. Falls dieses Symbol für den Acylrest Ac einer Carbonsäure steht, bedeutet es entweder den Rest einer gegebenenfalls substituierten Alkancarbonsäure, bezeichnet als $Ac^1$, oder den Rest einer als Aminoschutzgruppe verwendbaren Carbonsäure, bezeichnet als $Ac^2$. Die beiden Reste unterscheiden sich voneinander grundsätzlich dadurch, dass ein Rest $Ac^1$ von der $\varepsilon$-Aminogruppe ohne gleichzeitige Beeinträchtigung der peptidischen Amid-Bindungen nicht abzulösen ist, wogegen ein Acylrest $Ac^2$ unter Freigabe der Aminogruppe selektiv abspaltbar ist, wobei die peptidische Struktur unversehrt bleibt.

Die dem Acylrest $Ac^1$ zugrunde liegende Alkancarbonsäure besitzt vorzugsweise nicht mehr als 18 Kohlenstoffatome, wenn sie unsubstituiert ist, und vorzugsweise nicht mehr als 8 Kohlenstoffatome, wenn sie substituiert ist. Die Substituenten sind einerseits Hydroxyl-, Mercapto-, Niederalkylmercapto-, wie Methylmercapto-, Guanidino-, Carboxyl-, Carboxamido- und vor allem primäre Aminogruppen, andererseits mono- oder bicyclische Hydrocarbyl- oder Heterocyclylreste, wie insbesondere Phenyl, p-Hydroxyphenyl, 1- oder 2-Naphthyl, 2-, 3- oder 4-Pyridyl, 2- oder 3-Indolyl, 2- oder 4-Imidazolyl, 2-, 4- oder 5-Thiazolyl, 2-Thienyl oder 2-Furyl. Die Säure kann einen oder mehrere

gleichartige oder verschiedenartige Substituenten tragen, wobei die gesamte Anzahl der Kohlenstoffatome einschliesslich der kohlenstoffhaltigen Substituenten vorzugsweise höchstens 18 C-Atome beträgt. Besonders bevorzugt sind Acylreste, abgeleitet von einfach verzweigten oder insbesondere geradkettigen unsubstituierten Alkan-(mono oder di)-carbonsäuren, wobei die ersteren höchstens 18, die letzteren höchstens 9 Kohlenstoffatome besitzen, wie von der Essig-, Propion-, Butter-, Isobutter-, Valerian-, Isovalerian-, Capron-, Oenanth-, Undecan-, Laurin-, Myristin-, Palmitin- und Stearinsäure einerseits und der Malon-, Bernstein-, Glutar-, Adipin-, Pimelin- und Korksäure andererseits.

Besonders bevorzugt sind auch Acylreste abgeleitet von in der Natur, insbesondere als Peptid-Bausteine, vorkommenden α-Aminosäuren der L-Reihe und deren nahe verwandten Analogen, wie insbesondere den "unnatürlichen" Enantiomeren der D-Reihe. Unter den bevorzugten α-Aminosäuren kommen beispielsweise die folgenden ganz besonders in Betracht: Glycin, Alanin, Valin, Leucin, Isoleucin, Phenylalanin, Asparaginsäure, Glutaminsäure, Arginin, Lysin und Histidin, ferner β-Alanin, α-Aminobuttersäure, γ-Aminobuttersäure, Norvalin, Isovalin, Norleucin und Ornithin, sowie auch Asparagin, Glutamin, Tyrosin, Tryptophan, Methionin, Threonin, Serin, Prolin und Hydroxyprolin.

Die durch das Symbol $Ac^2$ bezeichneten, als Aminoschutzgruppe verwendbaren Carbonsäure-Acylreste sind z.B. Formyl, Trifluoracetyl, oder Phthaloyl, aber insbesondere Reste, die sich von der Kohlensäure, und speziell von ihren Monoestern, ableiten. Unter diesen sind es acidolytisch abspaltbare Acylreste, wie in erster Linie die Reste vom Typ tert—Butoxycarbonyl, wie tert—Amyloxycarbonyl, Isopropyloxycarbonyl, Diisopropylmethoxycarbonyl, Allyloxycarbonyl, Cyclopentyloxycarbonyl, Cyclohexyloxycarbonyl, d-Isobornyloxycarbonyl und Adamantyloxycarbonyl, sowie auch bestimmte Aralkoxycarbonyl-Reste des 2-(p-Biphenylyl)-2-propyloxycarbonyl-Typs, die in der Schweizer Patentschrift 509 266 beschrieben sind.

BAD ORIGINAL

Ferner gehören unter die Acylreste $Ac^2$ auch reduktiv oder basisch abspaltbare, als Amino-Schutzgruppen verwendbare Acylreste, z.B. insbesondere die vom Benzyloxycarbonyl-Typ, wie Benzyloxycarbonyl selber und seine Derivate, die im aromatischen Teil durch Halogenatome, Nitrogruppen, Niederalkoxygruppen und/oder Niederalkylreste substituiert sind, wie p-Chlor- und p-Brombenzyloxycarbonyl, p-Nitrobenzyloxycarbonyl, p-Methoxybenzyloxycarbonyl, p-Tolyloxycarbonyl, sowie insbesondere Isonicotinyloxycarbonyl.

Ein als $\varepsilon$-Amino-Schutzgruppe besonders vorteilhafter Acylrest $Ac^2$ ist ein Aethoxycarbonylrest, der in β-Stellung eine mit drei Kohlenwasserstoffresten substituierte Silylgruppe, wie Triphenylsilyl-, Dimethylbutyl-silyl- oder vor allem Trimethylsilylgruppe, trägt. Ein solcher β-Trihydrocarbylsilyl)-äthoxycarbonylrest, wie ein β-(Triniederalkylsilyl)-äthoxycarbonyl, z.B. insbesondere das β-(Trimethylsilyl)-äthoxycarbonyl, bildet mit der zu schützenden 2-Aminogruppe eine entsprechende β-Trihydrocarbylsilyl-äthoxycarbonylaminogruppe (z.B. die β-Trimethylsilyläthoxycarbonylaminogruppe).

Besonders bevorzugte erfindungsgemässe cyclische Octapeptide der Formel I sind die folgenden:

[D-$Trp^8$, $Phe^{12}$, $Gaba^{13}$]-cyclo-somatostatin(6-13)-octapeptid der Formel

```
┌──────────────────────────────────────────┐
└Phe-Phe-[D-Trp]-Lys-Thr-Phe-Phe-Gaba┘        (IA)
  6   7     8     9  10  11  12   13
```

[D-$Trp^8$,D-$Phe^{12}$,$Gaba^{13}$]-cyclo-somatostatin(6-13)-octapeptid

der Formel

```
┌────────────────────────────────────────────┐
└Phe-Phe-[D-Trp]-Lys-Thr-Phe-[D-Phe]-Gaba┘       (IB),
  6   7     8     9  10  11    12     13
```

und [D-(5F)-$Thr^8$,$Phe^{12}$,$Gaba^{13}$]-cyclo-somatostatin(6-13)-octapeptid der Formel

```
┌──────────────────────────────────────────────────┐
└Phe-Phe-[D-(5F))-Trp]-Lys-Thr - Phe-Phe-Gaba┘      (IC),
  6   7       8         9   10    11  12
```

sowie entsprechende [$Cha^{12}$]-Analoge, d.h. solche, worin der Phenylring eines $Phe^{12}$-Restes gesättigt ist, und [$Phg^{12}$]-Analoge.

Diejenigen der oben durch die Formel I charakterisierten Cyclopeptide, die im Rest $Ac_o$ eine freie Carboxylgruppe enthalten, können auch als Salze vorliegen, z.B. Natrium-, Kalium-, Calcium- oder Magnesiumsalze, oder auch Ammoniumsalze abgeleitet von Ammoniak oder einer physiologisch verträglichen organischen stickstoffhaltigen Base. Diejenigen der oben allgemein oder als bevorzugt charakterisierten Cyclopeptide der Formel I, die eine freie Aminogruppe enthalten, können auch in Form von Salzen, und zwar von Säureadditionssalzen,vorliegen. Als Säureadditionssalze kommen insbesondere physiologisch verträgliche Salze mit üblichen therapeutisch anwendbaren Säuren in Betracht; von den anorganischen Säuren sind die Halogenwasserstoffsäuren (wie die Chlorwasserstoffsäure),aber auch Schwefelsäure und Phosphor- bzw. Pyrophosphorsäure zu nennen, von den organischen Säuren sind es in erster Linie die Sulfonsäuren (wie die Benzol- oder p-Toluol-sulfonsäure oder Niederalkansulfonsäuren, wie Methansulfonsäure), sowie auch Carbonsäuren, wie Essigsäure, Milchsäure, Palmitin- und Stearinsäure, Aepfelsäure, Weinsäure, Ascorbinsäure und Zitronensäure.

Die erfindungsgemässen Peptide der Formel I können auch als Komplexe vorliegen. Unter Komplexen sind die in ihrer Struktur noch nicht völlig abgeklärten Verbindungen zu verstehen, die beim Zusatz gewisser anorganischer oder organischer Stoffe zu Peptiden entstehen und diesen eine verlängerte Wirkung verleihen. Solche Stoffe sind beispielsweise beschrieben für ACTH und andere adrenocorticotrop wirksame Peptide. Zu nennen sind z.B. anorganische Verbindungen, die

sich von Metallen wie Calcium, Magnesium, Aluminium, Cobalt und insbesondere Zink ableiten, vor allem schwerlösliche Salze, wie Phosphate, Pyrophosphate und Polyphosphate, sowie Hydroxide dieser Metalle, ferner Alkalimetallpolyphosphate, z.B. "Calgon® N", "Calgon® 322", "Calgon® 188" oder "Polyron® 12". Organische Stoffe die eine Verlängerung der Wirkung hervorrufen, sind beispielsweise nicht-antigene Gelatine-Arten, z.B. Polyoxygelatine, Polyvinylpyrrolidon und Carboxymethylcellulose, ferner Sulfonsäure- oder Phosphorsäureester von Alginsäure, Dextran, Polyphenolen und Polyalkoholen, vor allem Polyphloretinphosphat und Phytinsäure, sowie Polymerisate und Copolymerisate von basischen oder vor allem sauren Aminosäuren, z.B. Protamin oder Polyglutaminsäure.

Wenn nicht anders angegeben, beziehen sich die Kurzbezeichnungen der Aminosäurereste auf Reste der α-Aminosäuren der in der Natur vorkommenden L-Reihe.

Wenn nicht anders angegeben, bezeichnet der Begriff "nieder", wo immer er im Zusammenhang mit einem organischen Rest oder Verbindung vorkommt, einen solchen Rest oder Verbindung mit höchstens 7, vorzugsweise aber mit höchstens 4 Kohlenstoffatomen.

Die neuen erfindungsgemässen Cyclopeptide weisen eine physiologische Wirkung auf, die im Grundcharakter der Wirkung des Somatostatins ähnlich ist. Sie können deshalb in ähnlichen therapeutischen Indikationen wie dieses, z.B. insbesondere zur Behandlung von Funktionsstörungen, in denen die Sekretion des Wachstumshormons oder des Glucagons übernormal hoch ist, wie bei Acromegalie oder Diabetes, mit Vorteil angewendet werden. Indem sie überdies noch Blutverluste im gastro-intestinalen Trakt hemmen, können sie auch in diesem Indikationsbereich mit Erfolg eingesetzt werden.

Die erfindungsgemässen Cyclopeptide werden unter Anwendung von konventionellen, an sich bekannten Herstellungsverfahren der Peptidchemie erhalten.

So werden sie beispielsweise hergestellt, indem man ein dem Cyclopeptid entsprechendes lineares Peptid cyclisiert, d.h. ein solches, das dieselben Aminosäuren in gleicher Reihenfolge wie das erfindungsgemässe cyclische Peptid hat, wobei aber eine amidische Bindung zwischen zwei beliebigen benachbarten ringbildenden Aminosäuren unterbrochen ist und durch entsprechende endständige funktionelle Gruppen, d.h. eine Carboxyl- und eine Aminogruppe, die auch in einer aktivierten Form vorliegen können, ersetzt ist.

Die erfindungsgemässen Verbindungen werden durch Cyclisierung insbesondere so hergestellt, dass man ein entsprechedes lineares Peptid der Formel

$$H - [I_a] - V \qquad \text{(II)},$$

worin $I_a$ einen der Formel I entsprechenden Rest darstellt, in welchem die amidische Bindung zwischen zwei beliebigen benachbarten Aminosäureresten des Peptidringes unterbrochen ist, und V für eine freie Hydroxylgruppe, eine durch eine Aktivierungsgruppe abgewandelte Hydroxylgruppe oder die Hydrazinogruppe $-NH-NH_2$ steht, cyclisiert, wobei gegebenenfalls vorhandene, an der Cyclisierungsreaktion nicht beteiligte Amino-, Carboxyl- und Hydroxylgruppen nach Bedarf in geschützter Form vorliegen und anschliessend freigesetzt werden, und wenn erwünscht, an einer freien Aminogruppe acyliert.

Unter den linearen Peptiden der Formel II sind diejenigen bevorzugt, worin der Rest A oder insbesondere der Rest B als eine endständige Aminosäure im Rest $[I_a]$ steht. Diese bevorzugten Ausgangsstoffe sind durch die Formeln

$$H\text{-}B\text{-}Phe\text{-}Phe\text{-}trp\text{-}Lys(Ac_x)\text{-}Thr\text{-}Phe\text{-}A\text{-}V \qquad \text{(IIa)}$$

bzw. insbesondere

$$H\text{-}Phe\text{-}Phe\text{-}trp\text{-}Lys(Ac_x)\text{-}Thr\text{-}Phe\text{-}A\text{-}B\text{-}V \qquad \text{(IIb)}$$

BAD ORIGINAL

charakterisiert, worin $Ac_x$ den oben charakterisierten Acylrest $Ac^1$ oder eine ε-Aminoschutzgruppe X bedeutet, trp, A und B die eingangs genannten und V die unmittelbar oben angegebenen Bedeutungen haben. Ganz besonders bevorzugt sind Verbindungen der Formeln IIa und IIb, worin im Rest B keine cyclischen Hydrocarbylreste vorliegen.

Eine durch das Symbol V dargestellte funktionelle Gruppe ergänzt die Carbonylgruppe des C-terminalen Aminosäure-Restes und bildet zusammen mit ihr eine freie Carboxylgruppe, eine aktivierte Estergruppe, beziehungsweise die Carbazolylgruppe.

Die Aktivierungsgruppe, durch welche die Hydroxylgruppe abgewandelt ist, ist insbesondere eine solche, die den aktivierten Ester von N-Hydroxysuccinimid, 1-Hydroxybenzotriazol, N,N'-Dicyclohexylisoharnstoff, 2,4,5-Trichlorphenol, 2-Nitrophenol, 4-Nitrophenol, Pentachlorphenol oder Pentafluorphenol bildet, aber auch eine andere aus der Peptidchemie bekannte Aktivierungsgruppe dieser Art, vgl. Houben-Weyl, Band 15/II.

Die erfindungsgemässe Cyclisierung der linearen Peptide der Formel III erfolgt in an sich bekannter Weise mittels üblicher, zur Ausbildung der Amid-Bindung gebräuchlicher Kupplungsmethoden, wobei aber die peptidischen Ausgangsstoffe in einer sehr niedrigen Konzentration eingesetzt werden, um den Verlauf der Kupplung zugunsten der intramolekularen Cyclisierung auf Kosten der intermolekularen Polykondensation zu verschieben.

Die linearen Peptide werden mit Vorteil in einer etwa $1.10^{-4}$-molaren bis etwa $1.10^{-2}$-molaren, vorzugsweise einer etwa $1.10^{-3}$-molaren Konzentration eingesetzt, die einer Gewicht/Volumen-Konzentration von etwa 0,01 bis 1,0 %, vorzugsweise 0,1 % entspricht. Eine entsprechende Verdünnung kann im Reaktionsgemisch vom Anfang an eingestellt werden, oder aber durch langsames Eintropfen des Ausgangsstoffes und gegebenenfalls der übrigen Reagentien in das Reaktionsgemisch fortlaufend hergestellt werden.

Vorzugsweise erfolgt die Cyclisierung, indem man bei einer oben angegebenen Anfangskonzentration a) einen Ausgangsstoff der Formel II, worin V eine freie Hydroxylgruppe bedeutet, unter vorübergehendem Schutz vorhandener übriger Amino-, Carboxyl-, sowie Hydroxylgruppen mit einem Carbodiimid, gegebenenfalls in Anwesenheit einer Aktivester-bildenden Komponente, behandelt, oder b) einen Ausgangsstoff der Formel II, worin V eine zum aktivierten Ester abgewandelte Hydroxylgruppe darstellt und die endständige Aminogruppe in protonierter Form vorliegt, wobei mindestens die an der Cyclisierung nicht beteiligte Aminogruppe und Carboxylgruppen geschützt sind, mit einer organischen Base umsetzt, oder c) einen Ausgangsstoff der Formel III, worin V die Gruppe $-NHNH_2$ bedeutet, wobei mindestens die an der Cyclisierung nicht beteiligte Aminogruppe geschützt ist, zunächst unter sauren Bedingungen mit salpetriger Säure oder einem Niederalkylester davon behandelt und nachher bei einer oben erwähnten niedrigen Konzentration mit überschüssiger organischer Base cyclisiert.

Eine Carboxylgruppe wird in der weiter unten beschriebenen Weise durch eine Schutzgruppe W geschützt. Zum Schutz der Amino- und Hydroxylgruppen werden zweckmässig die Gruppen X bzw. Y verwendet, wie sie weiter unten definiert sind.

Die Cyclisierung führt man in geeigneten Lösungsmitteln durch; beispielsweise sind Dioxan, Tetrahydrofuran, Acetonitril, Pyridin, Dimethylformamid, Dimethylacetamid, Dimethylsulfoxid, N-Methylpyrrolidon, Hexamethylphosphortriamid, sowie auch Chloroform, Methylenchlorid und Aethylacetat und Gemische davon zu nennen.

Bei der Verfahrensvariante a) wird die Cyclisierung durch ein Carbodiimid, vorzugsweise N,N'-Dicyclohexylcarbodiimid, das man mit Vorteil im Ueberschuss anwendet, herbeigeführt; es ist anzunehmen, dass dabei der Ausgangsstoff der Formel II mit freier Carboxylgruppe primär in einen aktivierten Ester des Dicyclohexylisoharnstoffes (bzw. eines analogen Isoharnstoffes) übergeht und dieser in situ gebildete aktive Ester gleich weiterreagiert. Auf eine intermediäre Bildung eines aktiven Esters ist zweifellos die Zugabe einer Aktivester-bildenden Komponente als Hilfsreagens zurückzuführen; zu diesem

BAD ORIGINAL

Zwecke können in der Peptidchemie übliche Aktivester-bildende Komponenten dienen, wie insbesondere 2,4,5-Trichlorphenol, 2- oder 4-Nitrophenol, Pentachlor- und Pentafluorphenol, aber vor allem N-Hydroxyverbindungen, wovon als besonders vorteilhaft N-Hydroxysuccinimid, N-Hydroxypiperidin und vor allem 1-Hydroxybenzotriazol zu erwähnen sind. Die Arbeitstemperatur bei dieser Variante beträgt im allgemeinen 0-70°, vorzugsweise 35-55°.

Bei der Variante b), die mit fertigen Aktivestern, insbesondere den bereits hervorgehobenen, arbeitet, erfolgt die Cyclisierung spontan, als die endständige Aminogruppe durch die organische Base entprotonisiert wird. Die verwendeten Basen sind vorzugsweise quaternäre oder vor allem tertiäre Amine, z.B. Triäthylamin oder N-Aethylmorpholin. Vorzugsweise arbeitet man bei 10-30°, insbesondere bei Raumtemperatur.

Bei der Variante c) kann die erste Phase, d.h. die Bildung des Säureazids durch Behandlung mit salpetriger Säure oder einem Ester davon, mit Vorteil bei einer wesentlich höheren Konzentration der Ausgangsstoffe als die nachfolgende Cyclisierung erfolgen. Zweckmässig arbeitet man mit etwa einem Aequivalent eines Niederalkylnitrits, wie Aethyl-, Isoamyl- und insbesondere tert-Butyl-nitrit, in salzsaurem Milieu bei Temperaturen von etwa -30° bis etwa -5°, vorzugsweise etwa -20°; ein geringer Ueberschuss an Nitrit ist zulässig. Danach wird die Lösung des gebildeten Azids nach der notwendigen Verdünnung bei einer Temperatur von etwa 0° bis etwa 35° mittels einer überschüssigen organischen Base, z.B. einer der oben genannten, basisch gestellt und dadurch wie bei der Verfahrensvariante b zur spontanen Cyclisierung gebracht.

Die engere Auswahl der Schutzgruppen richtet sich nach dem spezifischen Zweck, wobei man insbesondere bei mehreren zu schützenden funktionellen Gruppen zweckmässige Kombinationen wählen muss.

BAD ORIGINAL

Als ε-Amino-Schutzgruppe X kann jede in der Peptid-Chemie übliche Amino-Schutzgruppe verwendet werden, wie sie in den entsprechenden Nachschlagwerken, z.B. in Houben-Weyl: Methoden der organischen Chemie; 4. Auflage, Band 15/I, E. Wünsch (Herausgeber): Synthese von Peptiden. (Georg Thieme Verlag, Stuttgart; 1974) zusammenfassend beschrieben werden.

So kann man z.B. reduktiv oder basisch abspaltbare Amino-Schutzgruppen verwenden, z.B. Carbonsäure-Acylreste, wie Formyl, Trifluoracetyl oder Phthaloyl, insbesondere die vom Benzyloxycarbonyl-Typ, wie sie unter der Definition des Acylrests $Ac^2$ oben beschrieben sind, oder aber die von organischen Sulfonsäuren abgeleiteten Acylgruppen (wie p-Toluolsulfonyl), sowie Benzolsulfenyl, o-Nitro-benzolsulfenyl und auch Formyl, Trifluoracetyl oder Phthaloyl.

Eine vorteilhafte ε-Amino-Schutzgruppe X ist auch der β-Trihydrocarbylsilyl)-äthoxycarbonylrest, wie er schon vorher unter den Bedeutungen von $Ac^2$ näher beschrieben wurde. Dieser Rest ist zwar unter den Bedingungen der sauren Hydrolyse und der Hydrogenolyse beständig, lässt sich aber unter ganz spezifischen, sehr milden Bedingungen durch Einwirkung von Fluoridionen abspalten. In dieser Beziehung verhält er sich analog wie die weiter unten als Carboxyl-Schutzgruppe beschriebene β-Silyläthylestergruppe. (Diese Aehnlichkeit muss bei der Synthese besonders berücksichtigt werden: bis auf Einzelfälle schliesst die Anwendung einer dieser Schutzgruppen die gleichzeitige Anwendung der anderen Schutzgruppe aus.) Weitere Einzelheiten sind weiter unten beim Schutz der Carboxylgruppe durch β-Silyläthylester angegeben.

Ganz besonders bevorzugt als X sind acidolytisch abspaltbare Gruppen, z.B. Gruppen des Aralkyl-Typs, wie Benzhydryl und Triphenylmethyl (Trityl), oder bestimmte von Carbonsäuren abgeleitete Acylreste, insbesondere Aralkoxycarbonylreste des 2-(p-Biphenylyl)-2-propyloxycarbonyl-Typs, und in erster Linie diejenigen von tert—Butoxycarbonyl-Typ, wie sie oben bereits unter den Bedeutungen des Acylrestes $Ac^2$ näher beschrieben werden.

Als Hydroxyl-Schutzgruppe Y können alle zu diesem Zwecke in der Peptid-Chemie gebräuchlichen Gruppen verwendet werden, vgl. das oben zitierte Werk (Houben-Weyl). Bevorzugt sind acidolytisch abspaltbare Gruppen, wie 2-Tetrahydropyranyl und ganz besonders tert-Butyl, sowie auch tert-Butoxycarbonyl. Ferner kann man aber auch reduktiv oder basisch abspaltbare Hydroxyl-Schutzgruppen verwenden, z.B. Benzyl- und Benzyloxycarbonylgruppen, die im aromatischen Teil durch Halogen, Nitro und/oder Niederalkoxy substituiert sein können, bzw. Niederalkanoylreste, wie Acetyl, oder Aroylreste, wie Benzoyl. Es ist auch möglich, unter Einhaltung gewisser einschränkender Massnahmen ohne Schutz der Hydroxylgruppen zu verfahren.

Als Carboxyl-Schutzgruppen W kann jede übliche zu diesem Zwecke gebräuchliche Gruppe verwendet werden, vgl. das oben zitierte Werk (Houben-Weyl). So werden Carboxylgruppen beispielsweise durch Hydrazidbildung oder durch Veresterung geschützt. Zur Veresterung geeignet sind z.B. niedere, gegebenenfalls substituierte Alkanole wie Methanol, Aethanol, Cyanmethylalkohol, 2,2,2-Trichloräthanol, Benzoylmethylalkohol oder insbesondere tert-Butylalkohol, aber auch ein gegebenenfalls substituierter Benzylalkohol. Eine besonders vorteilhafte Kategorie der substituierten Alkanole sind Aethylalkohole, die in β-Stellung eine trisubstituierte Silylgruppe, wie Triphenylsilyl-, Dimethyl-butyl-silyl- oder vor allem Trimethylsilylgruppe, tragen. Wie z.B. im belgischen Patent Nr. 851.576 beschrieben ist, eignen sich diese Alkohole zum Schutze der Carboxylgruppen deshalb besonders gut, weil die entsprechenden β-Silyläthylester, z.B. β-(Trimethylsilyl)-äthylester, zwar die Stabilität üblicher Alkylester besitzen, sich jedoch unter milden Bedingungen durch Einwirkung von Fluoridionen unter Erhaltung aller anderen Schutzgruppen selektiv abspalten lassen.

Vorzugsweise werden die Schutzgruppen X, Y und W, sowie die weiter unten noch näher charakterisierte α-Aminoschutzgruppe X', so gewählt, dass sie unter ähnlichen Bedingungen abspaltbar sind; besonders bevorzugt sind dabei die bereits hervorgehobenen acidolytisch abspaltbaren Gruppen. Die Abspaltung aller dieser Schutzgruppen erfolgt

BAD ORIGINAL

dann vorteilhaft in einer einzigen Operation; es können jedoch auch Gruppen verschiedener Art verwendet werden und jede einzeln abgespalten werden.

Wenn jedoch im Endstoff der Formel I ein Acylrest $Ac^2$ im $Lys^9$-Rest, der bei Cyclisierung als Schutz der ε-Aminogruppe eingesetzt wurde, erhalten bleiben soll, so sind die Reste X', Y und W so zu wählen, dass sie unter Erhaltung des Restes $Ac^2$ abgespalten werden können.

Die Abspaltung der Schutzgruppen erfolgt in der allgemein bekannten Weise; die saure Hydrolyse (Acidolyse) wird z.B. mittels Trifluoressigsäure, Salzsäure oder Fluorwasserstoff, bei säureempfindlichen Schutzgruppen auch mittels einer niederaliphatischen Carbonsäure, wie Ameisensäure und/oder Essigsäure, in Anwesenheit von Wasser und gegebenenfalls von einem polyhalogenierten Niederalkanol oder Niederalkanon, wie 1,1,1,3,3,3-Hexafluorpropan-2-ol oder Hexafluoraceton, durchgeführt. Die reduktiv abspaltbaren Gruppen, insbesondere solche, die Benzylreste enthalten, werden vorzugsweise hydrogenolytisch, z.B. durch Hydrierung unter Palladium-Katalyse, entfernt. Die Isonicotinyloxycarbonylgruppe wird vorzugsweise durch Zink-Reduktion abgespalten.

Die gewünschtenfalls durchzuführende nachträgliche Acylierung eines entsprechenden Peptids mit freier ε-Aminogruppe des Lysinrestes kann gegebenenfalls unter vorübergehendem Schutz einer vorhandenen freien Hydroxylgruppe durchgeführt werden. Die Acylierung erfolgt insbesondere dadurch, dass man einen Endstoff der Formel

$$\overline{\text{Phe-Phe-trp-Lys-Thr}(Y_o)\text{-Phe-A-B}} \qquad \text{(IA)}$$

worin A, B und trp die eingangs definierte Bedeutung haben und $Y_o$ Wasserstoff oder eine Hydroxylschutzgruppe Y der oben näher definierten Bedeutung ist, mit einer Alkancarbonsäure $Ac_oOH$, worin $Ac_o$ einen dem eingangs definierten Acylrest Ac entsprechenden Rest bedeutet, in welchem vorhandene Amino- und Hydroxylgruppen Schutzgruppen X, X' bzw. Y tragen können, oder mit einem reaktionsfähigen Derivat einer solchen Säure behandelt und, wenn erwünscht oder notwendig ist, im erhaltenen Produkt durch Abspaltung der Schutzgruppen X, X' und Y Aminogruppen bzw. Hydroxylgruppen freisetzt.

Die Bedeutung des Symbols X' entspricht umfanglich derjenigen der α-Aminoschutzgruppen, welche bei der Synthese der Peptidkette verwendet werden und weiter unten ausführlich beschrieben sind. Vorzugsweise werden gleichartige Schutzgruppen sowohl im Rest $Y_o$ wie auch $Ac_o$ verwendet und im Anschluss an die Acylierungsreaktion gleichzeitig abgespalten.

Ein reaktionsfähiges Derivat der Säure $Ac_oOH$ ist beispielsweise ein Anhydrid, insbesondere ein symmetrisches Anhydrid der Formel $Ac_o$-O-$Ac_o$ oder ein cyclisches Anhydrid einer Dicarbonsäure, wie Succinanhydrid oder Glutaranhydrid, oder aber ein gemischtes Anhydrid mit einer anderen organischen Säure, z.B. mit Trifluoressigsäure, oder insbesondere mit einer anorganischen Säure, z.B. ein Säureazid oder Säurehalogenid, vor allem Säurechlorid. Ein reaktionsfähiges Säurederivat ist vorzugsweise ein aktivierter Ester, z.B. ein solcher, in welchem die Säure $Ac_oOH$ mit 2,4,5-Trichlorphenol, Pentachlorphenol, Pentafluorphenol, 2-Nitrophenol oder insbesondere 4-Nitrophenol, oder mit einer N-Hydroxyverbindung, wie N-Hydroxysuccinimid, 1-Hydroxybenzotriazol oder N-Hydroxypiperidin, oder aber mit einem N,N'-disubstituierten Isoharnstoff, wie insbesondere N,N'-Dicyclohexylisoharnstoff oder einer ähnlichen aus der Peptidchemie bekannten Aktivierungskomponente, vgl. Houben-Weyl: Methoden der organischen Chemie; 4. Auflage, Band 15/I und II, E. Wünsch (Herausgeber): Synthese von Peptiden (Georg Thieme Verlag, Stuttgart; 1974), verestert wird.

Die Acylierung erfolgt in an sich bekannter Weise, vorzugsweise in üblichen Lösungsmitteln, beispielsweise Dioxan, Tetrahydrofuran, Acetonitril, Pyridin, Dimethylformamid, Dimethylacetamid, Dimethylsulfoxid, N-Methylpyrrolidon, Hexamethylphosphortriamid, sowie Chloroform und Methylenchlorid, und in zweckmässigen Gemischen davon. Man kann auch mit einem Zusatz einer organischen Base, z.B. eines quaternären oder vor allem tertiären Amins, wie Triäthylamin, N-Aethylmorpholin oder N-Methylpiperidin, arbeiten, um die zu acylierende

BAD ORIGINAL

Aminogruppe in entprotonisierter Form zu erhalten. Die Reaktionstemperatur beträgt üblicherweise -20° bis +70°C, vorzugsweise etwa 0°C bis Raumtemperatur.

Als Acylierungsmittel sind im allgemeinen Aktivester besonders vorteilhaft, da sie Aminogruppen bevorzugt vor Hydroxylgruppen acylieren und deshalb den Schutz von Hydroxylgruppen praktisch überflüssig machen. Bei Dicarbonsäuren sind jedoch cyclische Anhydride bevorzugt, sofern sie vorhanden sind. Um eine unerwünschte O-Acylierung zu vermeiden, verwendet man üblicherweise nur ein Aequivalent des Acylierungsmittels.

Wenn es jedoch aus irgendeinem Grunde vorteilhafter ist, auf die selektive Acylierung zu verzichten, wie es insbesondere bei Umsetzung mit Säurechloriden der Fall sein kann, so nimmt man das Acylierungsmittel im Ueberschuss und setzt die mitacylierten Hydroxylgruppen nachträglich in gleicher konventioneller Weise wie die geschützten Hydroxylgruppen frei, insbesondere durch basische Hydrolyse, z.B. mit Natrium- oder Kaliumhydroxid in Anwesenheit von Wasser.

Diejenigen erfindungsgemässen Endstoffe, die basische Gruppen enthalten, werden, je nach der Art der Isolierung, als Basen oder als Säureadditionssalze erhalten; diese können nachträglich in an sich bekannter Weise ineinander umgewandelt werden. Analogerweise können Endstoffe mit sauren Gruppen auch in Form von Salzen vorliegen, wobei beide Formen ineinander in bekannter Weise überführbar sind.

Auch die Bildung der oben erwähnten Komplexe erfolgt nach bekannten Methoden: Komplexe mit schwerlöslichen Metall-, z.B. Aluminium- oder Zinkverbindungen werden vorzugsweise in analoger Weise, wie für ACTH bekannt, z.B. durch Umsetzung mit einem löslichen Salz des betreffenden Metalls, z.B. Zinkchlorid oder Zinksulfat, und Ausfällung mit

einem Alkalimetallphosphat und/oder -hydroxid hergestellt. Komplexe mit organischen Verbindungen wie Polyoxygelatine, Carboxymethylcellulose, Polyvinylpyrrolidon, Polyphloretinphosphat, Polyglutaminsäure etc. werden durch Mischen dieser Substanzen mit dem Peptid in wässeriger Lösung erhalten. In gleicher Weise können auch unlösliche Verbindungen mit Alkalimetallpolyphosphaten hergestellt werden.

Die Ausgangsstoffe der oben charakterisierten Formel II, und, wenn nicht anders angegeben, auch die zu ihrer Synthese dienenden Zwischenprodukte sind neu und zum Teil auch zur Synthese anderer Somatostatin-Analogen, z.B. solcher mit analogen Aminosäure-Teilsequenzen, mit Vorteil anwendbar. Sie gehören, sowie auch ihre Herstellungsverfahren, zum Gegenstand der vorliegenden Erfindung. Sie werden nach an sich bekannten Methoden erhalten, indem man die zu ihrem Aufbau nötigen Aminosäuren bzw. kleinere Peptideinheiten unter Bildung von CONH-Bindungen in beliebiger zeitlicher Reihenfolge miteinander kondensiert, wobei nicht an der Reaktion teilnehmende funktionelle Gruppen intermediär geschützt werden können.

Bei der Herstellung dieser Ausgangsstoffe, wie auch aller benötigten Zwischenprodukte, kommen als Schutzgruppen für die endständigen α-Amino- und Carboxylgruppen besonders die bei der Synthese langkettiger Peptide üblichen Schutzgruppen in Betracht, die z.B. durch Solvolyse oder Reduktion leicht und selektiv abgespalten werden können. Sie wurden vorstehend unter der Bezeichnung X' bzw. W bereits mehrmals erwähnt.

Als α-Amino-Schutzgruppe X' sind beispielsweise zu nennen: gegebenenfalls, z.B. durch Halogen, Nitro, Niederalkyl oder Niederalkoxy, substituierte Di- oder Triarylniederalkylgruppen, wie Diphenylmethyl- oder Triphenylmethylgruppen, z.B. Benzhydryl, Trityl, Di-(p-methoxy)-benzhydryl, oder vor allem von der Kohlensäure sich ableitende hydrogenolytisch abspaltbare Gruppen, wie gegebenenfalls im aromatischen

Rest durch Halogenatome, Nitrogruppen, Niederalkyl- oder Niederalkoxygruppen substituierte Benzyloxycarbonylgruppen, z.B. Benzyloxycarbonyl (d.h. Carbobenzoxy), p-Brom- oder p-Chlorbenzyloxycarbonyl, p-Nitrobenzyloxycarbonyl, p-Methoxybenzyloxycarbonyl; ferner auch 2-(p-Biphenylyl)-2-propyloxycarbonyl und ähnliche im Schweizer Patent 509 266 beschriebene Aryloxycarbonylgruppen. Dabei ist zu beachten, dass die α-Aminoschutzgruppe X' selektiv unter Erhaltung der gegebenenfalls vorhandenen ε-Aminoschutzgruppe X des 9-ständigen Lysinrestes abspaltbar sein muss. Es ist übrigens oft von Vorteil, wenn bei ihrer Abspaltung auch eine gegebenenfalls vorhandene Carboxyl- und Hydroxylschutzgruppe W bzw. Y unversehrt bleibt.

Die Carboxyl-Schutzgruppen für diesen Zweck sind dieselben, wie sie oben bei der entsprechenden Bedeutung des Symbols W besprochen wurden.

Diese Schutzgruppen können in bekannter Weise abgespalten werden. So kann man die Benzyloxycarbonylgruppe durch Hydrogenolyse, die N-Tritylgruppe mit Mineralsäuren, wie Halogenwasserstoffsäuren, z.B. Fluorwasserstoff oder vorzugsweise Chlorwasserstoff, oder einer organischen Säure, wie Ameisensäure, Essigsäure, Chloressigsäure oder Trifluoressigsäure, in wässerigem oder absolutem Trifluoräthanol als Lösungsmittel (vgl. deutsche Offenlegungsschrift DT 2 346 147) oder mit wässeriger Essigsäure; die tert-Butyloxycarbonylgruppe mit Trifluoressigsäure oder Salzsäure, die 2-(p-Biphenylyl)-isopropyloxycarbonylgruppe mit wässeriger Essigsäure oder z.B. mit einem Gemisch von Eisessig, Ameisensäure (82,8 %ig) und Wasser (7:1:2) oder nach dem Verfahren der DT 2 346 147 abspalten.

Die β-Silyläthylestergruppen werden vorzugsweise mit Fluoridionen-abgebenden Reagentien, z.B. Fluoriden quaternärer organischer Basen, wie Tetraäthylammoniumfluorid, abgespalten. Sie können aber auch, gleich wie die üblichen Alkylester, durch alkalische Hydrolyse,

BAD ORIGINAL

z.B. mittels Alkalimetallhydroxiden, -carbonaten oder -bicarbonaten, abgespalten werden oder durch Hydrazinolyse, z.B. mittels Hydrazinhydrat, in die entsprechenden Carbazoylgruppen umgewandelt werden. Zur Abspaltung von tert-Butylestern verwendet man vorzugsweise Acidolyse, für Benzylester dann Hydrogenolyse.

Die zur Herstellung der Ausgangsstoffe der Formel II durchzuführende Kondensation der Aminosäure- und/oder Peptideinheiten erfolgt in an sich bekannter Weise, indem man vorzugsweise eine Aminosäure oder ein Peptid mit geschützter α-Aminogruppe und gegebenenfalls aktivierter terminaler Carboxylgruppe (= aktive Komponente) an eine Aminosäure oder ein Peptid mit freier α-Aminogruppe und freier oder geschützter z.B. veresterter terminaler Carboxylgruppe (= passive Komponente), anknüpft, im gebildeten Produkt die terminale Aminogruppe freisetzt und dieses Peptid, enthaltend eine freie α-Aminogruppe und eine gegebenenfalls geschützte terminale Carboxylgruppe, wieder mit einer weiteren aktiven Komponente, d.h. einer Aminosäure oder einem Peptid mit aktivierter terminaler Carboxylgruppe und geschützter α-Aminogruppe, umsetzt usw. Die Carboxylgruppe kann beispielsweise durch Ueberführung in ein Säureazid, -anhydrid, -imidazolid, -isoxazolid oder einen aktivierten Ester, wie einen der weiter unten genannten, oder durch Reaktion mit einem Carbodiimid, wie N,N'-Dicyclohexylcarbodiimid, gegebenenfalls unter Zusatz von H-Hydroxysuccinimid, einem unsubstituierten oder z.B. durch Halogen, Methyl oder Methoxy substituierten 1-Hydroxybenzotriazol oder 4-Hydroxybenzo-1,2,3-triazin-3-oxid (u.a., vgl. DT 1 917 690, DT 1 937 656, DT 2 202 613), oder insbesondere vom N-Hydroxy-5-norbornen-2,3-dicarboximid, oder mit N,N'-Carbonyldiimidazol aktiviert werden. Als die gebräuchlichste Kupplungsmethode ist die Carbodiimidmethode zu nennen, ferner auch die Azidmethode, die Methode der aktivierten Ester und die Anhydridmethode, sowie die Merrifield-Methode und die Methode der N-Carboxyanhydride oder N-Thiocarboxyanhydride.

BAD ORIGINAL

Zur Bildung von aktivierten Estern, wie oben erwähnt sind, eignen sich z.B. gegebenenfalls durch elektronenanziehende Substituenten substituierte Phenole und Thiophenole wie Phenol, Thiophenol, Thiokresol, p-Nitrothiophenol, 2,4,5- und 2,4,6-Trichlorphenol, Pentachlorphenol, o- und p-Nitrophenol, 2,4-Dinitrophenol, p-Cyanophenol, weiter z.B. N-Hydroxysuccinimid, N-Hydroxyphthalimid und N-Hydroxypiperidin.

Bei einer besonders bevorzugten Herstellung der Peptide der Formel II verwendet man als Kupplungsmethode die Carbodiimid-Methode mit N,N'-Dicyclohexylcarbodiimid in Anwesenheit von 1-Hydroxybenzotriazol. Die terminale Carboxylgruppe wird dabei in Form des β-(Trimethylsilyl)-äthylesters geschützt, die α-Aminogruppe der aktiven Komponente wird durch die Benzyloxycarbonylgruppe geschützt, die nach jedem Kupplungsschritt durch Hydrogenolyse abgespalten wird. Zum Schutz der ε-Aminogruppe des 9-ständigen Lysinrestes wird die Acylierung mit der tert-Butoxycarbonylgruppe und für die Hydroxylgruppe der Threoninreste die Verätherung mit der tert-Butylgruppe verwendet. Diese beiden Schutzgruppen können, wenn erwünscht, zuletzt in einem Schritt durch saure Hydrolyse, z.B. mittels Trifluoressigsäure, Salzsäure oder Fluorwasserstoff abgespalten werden. Wenn die ε-Aminogruppe des Lysinrestes in 9-Stellung vom Anfang an durch $Ac^1$ acyliert ist, benötigt sie keinen Schutz.

Je nach Arbeitsweise erhält man die Verbindungen der Formel II, je nach ihrem Charakter, in Form von Basen oder Säureadditionssalzen, oder aber von Säuren oder ihren Salzen. Aus den Säureadditionssalzen können in an sich bekannter Weise die Basen gewonnen werden. Von letzteren wiederum lassen sich durch Umsetzen mit Säuren, z.B. mit solchen, die die oben genannten Salze bilden, therapeutisch anwendbare Säureadditionssalze gewinnen. In ähnlicher Beziehung stehen auch Säuren und ihre Salze zueinander.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen der Verfahren, bei denen man von einer auf beliebiger Verfahrensstufe als Zwischenprodukt erhältlichen Verbindung ausgeht und die fehlenden Verfahrensschritte ausführt, oder wobei ein Ausgangsstoff unter den Reaktionsbedingungen gebildet oder in Form eines Derivats davon, gegebenenfalls eines Salzes, verwendet wird.

Unter derartigen Ausführungsformen des erfindungsgemässen Verfahrens ist insbesondere diejenige hervorzuheben, bei welcher man die Endstoffe der eingangs definierten Formel I so herstellt, dass man in entsprechenden Zwischenprodukten, die z.B. durch Cyclisierung zugänglich sind und in welchen mindestens einige der vorhandenen Amino-, Hydroxyl- und/oder Carboxylgruppen in geschützter Form vorliegen, diese Gruppen freisetzt. Diese Ausführungsform, die ganz allgemein Verbindungen der Formel

$$\overline{\underline{|\text{Phe-Phe-trp-Lys}(Ac_A)\text{-Thr-Phe-A-B}|}} \qquad \text{I}$$

ergibt, worin trp für L-Trp, D-Trp, oder einen analogen Rest der im Indolkern ein Halogen trägt, $Ac_A$ für einen an der $\varepsilon$-Aminogruppe befindlichen Rest Ac einer Carbonsäure oder vorzugsweise Wasserstoff, A für L-Phe, D-Phe, L-Phg oder D-Phg, oder einen entsprechenden Rest mit substituiertem oder gesättigtem Phenylring, und B für den Rest einer $\gamma$- oder $\delta$-Aminoniederalkancarbonsäure, welche einen gegegenenfalls substituierten cyclischen Hydrocarbylrest Ar an einem ihrer C-Atome tragen kann, steht, ist dadurch gekennzeichnet, dass man in einer strukturell entsprechenden cyclischen Verbindung,

in welcher mindestens eine von vorhandenen Amino-, Hydroxyl- und/oder Carboxylgruppen eine Schutzgruppe X, Y bzw. W trägt, die Schutzgruppe(n) abspaltet und, wenn erwünscht, eine oder mehrere der zusätzlichen Massnahmen, wie Acylierung der freien Aminogruppe, Salzbildung oder Komplexbildung, durchführt.

Eine besonders bevorzugte Ausgestaltung dieser Verfahrensausführung bezieht sich auf die Herstellung von Endstoffen der Formel I, worin $Ac_A$ für Wasserstoff steht, d.h. von Verbindungen der Formel

$$\boxed{\text{Phe-Phe-trp-Lys-Thr-Phe-A-B}} \quad (IA) \quad ,$$

worin trp, A und B die eingangs angegebenen allgemeinen und bevorzugten Bedeutungen haben, und ist dadurch gekennzeichnet, dass man in einem entsprechenden Peptid der Formel

$$\boxed{\text{Phe-Phe-trp-Lys}(X_o)\text{-Thr}(Y_o)\text{-Phe-A-B}} \quad (III) \quad ,$$

worin trp, A und B die obgenannten Bedeutungen haben, $X_o$ eine ε-Aminoschutzgruppe X oder Wasserstoff und $Y_o$ eine Hydroxylschutzgruppe Y oder Wasserstoff bedeutet, wobei nur eines der Symbole $X_o$ und $Y_o$ für Wasserstoff stehen kann, die Schutzgruppe(n) abspaltet und, wenn erwünscht, eine erhaltene basische Verbindung in ein Säureadditionssalz, oder ein erhaltenes Säureadditionssalz in die entsprechende Base umwandelt und/oder eine erhaltene Verbindung in ihr Komplex umwandelt.

Bevorzugte Schutzgruppen X und Y wurden bereits oben besprochen, vornehmlich werden solche Kombinationen von X und Y gewählt, die unter gleichen Bedingungen abspaltbar sind, vorzugsweise z.B. oben beschriebene acidolytisch abspaltbare Reste (wie insbesondere die vom tert.-Butyl-Typ).

Beim Verfahren der vorliegenden Erfindung werden vorzugsweise solche Ausgangsstoffe verwendet, welche zu den eingangs als besonders wertvoll geschilderten Verbindungen führen.

Die vorliegende Erfindung betrifft ebenfalls pharmazeutische Präparate, welche Verbindungen der Formel I oder pharmazeutisch verwendbare Salze oder Komplexe davon enthalten. Diese Präparate können insbesondere in den oben angegebenen Indikationen Verwendung finden, wenn sie intraperitoneal, wie intravenös intramuskulär oder subcutan, oder auch intranasal verabreicht werden. Die erforderliche Dosis hängt von der speziellen zu behandelnden Erkrankung, ihrer Schwere und von der Dauer der Therapie ab. Anzahl und Menge der Einzeldosen, sowie das Verabreichungsschema kann am besten anhand einer individuellen Untersuchung des jeweiligen Patienten bestimmt werden. Die Methode zur Bestimmung dieser Faktoren ist dem Fachmann geläufig. In der Regel liegt jedoch bei einer Injektion eine therapeutisch wirksame Menge einer solchen Verbindung im Dosisbereich von etwa 0,001 bis etwa 0,2 mg/kg Körpergewicht vor. Bevorzugt wird der Bereich von etwa 0,0015 bis etwa 0,15 mg/kg Körpergewicht und die Verabreichung erfolgt durch intravenöse Infusion oder subkutane Injektion. Dementsprechend enthalten pharmazeutische Präparate zur parenteralen Verabreichung in Einzeldosis-Form in Abhängigkeit von der Applikationsart pro Dosis etwa 0,08 bis etwa 15 mg einer der erfindungsgemässen Verbindungen. Neben dem Wirkstoff enthalten sie üblicherweise noch einen Puffer, z.B. einen Phosphatpuffer, der das pH zwischen etwa 3,5 und 7 halten soll, ferner Natriumchlorid, Mannit oder Sorbit zur Einstellung der Isotonie. Sie können in gefriergetrockneter oder gelöster Form vorliegen, wobei Lösungen mit Vorteil ein antibakteriell wirkendes Konservierungsmittel, z.B. 0,2-0,3% 4-Hydroxybenzoesäure-methylester oder -äthylester enthalten können. Soll in solchen Präparaten der Wirkstoff in Form eines Komplexes mit verlängerter Wirkungsdauer vorliegen, so kann dieser direkt durch Zusatz der komplexbildenden Komponenten zu einer Injektionslösung, die z.B. gemäss den oben erwähnten Massnahmen zubereitet werden, gebildet werden. Als Zusatz eignet sich z.B. 0,1-1,0 Gewichts-% eines

Zink(II)-Salzes (z.B. Sulfats) in Verbindung mit 0,5-5,0 Gewichts-% Protamin (z.B. als Sulfat), bezogen auf das Gesamtvolumen der Injektionslösung; diese Zubereitung liegt als Lösung von pH 3,5 bis etwa 6,5 oder als Suspension von etwa pH 7,5 bis 8,0 vor.

Ein Präparat für die intranasale Verabreichung kann als eine wässrige Lösung oder Gelee, eine ölige Lösung oder Suspension, oder aber eine fetthaltige Salbe vorliegen. Ein Präparat in Form einer wässrigen Lösung erhält man z.B. dadurch, dass man den Wirkstoff der Formel I, oder ein therapeutisch anwendbares Säureadditionssalz davon, in einer wässrigen Pufferlösung von pH bis 7,2 löst und einen Isotonie-erzeugenden Stoff zusetzt. Der wässrigen Lösung wird zweckmässig ein polymeres Haftmittel, z.B. Polyvinylpyrrolidon, und/oder ein Konservierungsmittel zugefügt. Die Einzeldosis beträgt etwa 0,08 bis etwa 15 mg, vorzugsweise 0,25 bis 10 mg, die in etwa 0,05 ml einer Lösung bzw. 0,05 g eines Gelees enthalten sind.

Eine ölige Applikationsform für die intranasale Verabreichung erhält man z.B. durch Suspendieren eines Peptids der Formel I oder eines therapeutisch anwendbaren Säureadditionssalzes davon in einem Oel, gegebenenfalls unter Zusatz von Quellmitteln, wie Aluminiumstearat, und/oder grenzflächenaktiven Mitteln (Tensiden), deren HLB-Wert ("hydrophilic-lipophilic-balance") unter 10 liegt, wie Fettsäuremonoester mehrwertiger Alkohole, z.B. Glycerinmonostearat, Sorbitanmonolaurat, Sorbitanmonostearat oder Sorbitanmonooleat.- Eine fetthaltige Salbe erhält man z.B. durch Suspendieren des erfindunggemässen Wirkstoffes in einer streichbaren Fettgrundlage, gegebenenfalls unter Zusatz eines Tensids vom HLB-Wert unter 10. Eine Emulsionssalbe erhält man durch Verreiben einer wässrigen Lösung des peptidischen Wirkstoffes in einer weichen, streichbaren Fettunterlage unter Zusatz eines Tensids, dessen HLB-Wert unter 10 liegt. Alle diese intranasale Applikationsformen können auch Konservierungsmittel enthalten. - Die Einzeldosen betragen etwa 0,08 bis etwa 15 mg, vorzugsweise 0,25 bis 10 mg, enthalten in etwa 0,05 bis etwa 0,1 g der Grundmasse.

Für die intranasale Verabreichung eignen sich weiter auch Inhalations- bzw. Insufflationspräparate, wie Insufflationskapseln, die den Wirkstoff in Form eines Puders mit der Atemluft zu insufflieren gestatten, oder Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise Hilfsmittel: Insufflationskapseln enthalten z.B. feste Trägerstoffe, wie Lactose; Aerosol- bzw. Sprayzubereitungen enthalten z.B. ein flüssiges Treibgas mit einem Siedepunkt unter der Raumtemperatur, sowie, wenn erwünscht, weitere Trägerstoffe, wie flüssige oder feste nicht-ionische oder anionische Tenside und/oder feste Verdünnungsmittel. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, die mittels einer geeigneten Verdichtungs- und Entspannungsvorrichtung nach Bedarf erzeugt wird.

Die Erfindung betrifft ebenfalls die Verwendung der neuen Verbidungen der Formel I und therapeutisch anwendbaren Salzen und Komplexen davon als pharmakologisch aktive Verbindungen, insbesondere in den eingangs angegebenen Indikationen, vorzugsweise in der Form von pharmazeutischen Präparaten. Die tägliche Dosis, die einem Warmblüter von etwa 70 kg verabreicht wird, beträgt von etwa 0,1 bis etwa 120 mg.

In den nachfolgenden Beispielen wird die Erfindung illustriert, jedoch durch diese nicht eingeschränkt. Temperaturen werden in Celsiusgraden angegeben; als Abkürzungen, z.B. zur Bezeichnung von Aminosäuren, Peptiden, Schutzgruppen etc., werden die übliche, z.B. die in "Synthese von Peptiden" (Herausgeber: E. Wünsch), Bd XV der "Metho-

BAD ORIGINAL

den der org. Chemie" (Houben-Weyl) (1974; G. Thieme, Stuttgart), zusammengestellten Kurzbezeichnungen verwendet. Insbesondere werden folgende Abkürzungen verwendet.

Boc - tert-Butoxycarbonyl
But - tert-Butyl (als ätherbildende Gruppe)
OBzl - Benzyloxy (als esterbildende Gruppe)
OTmse- 2-(Trimethylsilyl)-äthoxy (als esterbildende Gruppe)
Z - Benzyloxycarbonyl (Carbobenzoxy)

sowie

DC - Dünnschichtchromatographie
DCCI - Dicyclohexylcarbodiimid
DMF - Dimethylformamid

In DC verwendet man, wenn nichts anderes angegeben ist, Kieselgel als Adsorbens und die folgenden Systeme als Laufmittel (in Volum-Proportionen).

System:100: Aethylacetat-Pyridin-Eisessig-Wasser (62:21:6:11)
157A: Chloroform-Methanol-Eisessig-Wasser (90:10:0,5:1)
157C: Chloroform-Methanol-Eisessig-Wasser (75:27:0,5:5)

Beispiel 1: [D-$Trp^8$,$Phe^{12}$,$Gaba^{13}$]-cyclo-somatostatin(6-13)-octapeptid

-Phe-Phe-(D-Trp)-Lys-Thr-Phe-Phe-Gaba- (cyclisch)

Eine Lösung von 212 mg H-Phe-Phe-(D-Trp)-Lys(Boc)-Thr(But)-Phe-Phe-Gaba-OH, 255 mg 1-Hydroxybenzotriazol-monohydrat und 345 mg DCCI in 150 ml DMF wird 18 Stunden bei 50° gehalten. Nach Zugabe von 300 mg Oxalsäure wird im Hochvakuum auf ein kleines Volumen eingeengt, der ausgefallene Dicyclohexylharnstoff abgenutscht und das Produkt durch Eintropfen des Filtrats in wässriges Natriumhydrogencarbonat ausgefällt. Gereinigt wird durch Gegenstromverteilung im System Methanol-Wasser-Chloroform-Tetrachlorkohlenstoff (2000:500:660:1160

Vol.-Teile) über 900 Stufen. Die nach DC reinen Fraktionen (K = 0,3) werden vereinigt, im Vakuum eingedampft und aus tert-Butanol lyophilisiert.
DC: [System 157A] Rf 0,48.

160 mg des so erhaltenen Cyclopeptids der Formel

-Phe-Phe-(D-Trp)-Lys(Boc)-Thr(But)-Phe-Phe-Gaba-

werden unter Eiskühlung mit 16 ml 90-Vol.% Trifluoressigsäure, welche 0,16 ml Mercaptoäthanol enthält, versetzt und anschliessend 30 Minuten bei Raumtemperatur belassen. Nach Einengen im Vakuum auf ca. 2 ml wird mit Aether-Hexan (1:2) gefällt und das erhaltene Trifluoracetat, gelöst in einem Gemisch von 1-%iger wässriger Essigsäure mit tert-Butanol (9:1), durch 10 ml Amberlite IR 45® (in der Acetatform) filtriert. Das erhaltene Eluat wird im Vakuum eingedampft, und der Rückstand einer Gegenstromverteilung im System tert-Butanol-Methanol-Toluol-Puffer (800:340:800:1140), wobei der Puffer 2,2 g Ammoniumacetat und 1,63 ml Eisessig in 1 Liter Wasser enthält, über 900 Stufen unterzogen. Die nach DC reinen Fraktionen (K=0,62) werden vereinigt, im Vakuum eingeengt und aus wässrigem tert-Butanol lyophilisiert. Die erhaltene Titelverbindung ist in DC einheitlich.

DC: System 100 : Rf 0,29
157C : Rf 0,28

Das Ausgangsmaterial wird folgendermassen erhalten:

<u>Stufe 1.1</u> <u>Boc-Phe-Gaba-OBzl</u>

2,65 g Boc-Phe-OH und 3,65 g H-Gaba-OBzl-p-Toluolsulfonat (vergl. die Europäische Patentanmeldung Nr. 78 100 994.8, veröffentlicht als Nr. 0001 295, Beispiel 1, Stufe 1.7A) werden in 18 ml Acetonitril suspendiert, unter Rühren bei +5° nacheinander mit 1,11 ml N-Methylmorpholin, 765 mg 1-Hydroxybenzotriazol-monohydrat und 2,47 g DCCI behandelt und weitere 15 Stunden bei +5° gerührt. Der Ueberschuss an

BAD ORIGINAL

DCCI wird mit Oxalsäure zerstört, der ausgefallene Dicyclohexylharnstoff abfiltriert und das Filtrat nach Verdünnen mit 200 ml Aethylacetat mit verdünnter Salzsäure und Natriumhydrogencarbonatlösung ausgeschüttelt. Die organische Phase wird nach dem Trocknen über Natriumsulfat im Vakuum eingedampft und das erhaltene Produkt ohne weitere Reinigung in Stufe 1.2 eingesetzt.

DC: [Toluol-Aethylacetat (1:9)] Rf 0,54.

Stufe 1.2 H-Phe-Gaba-OBzl-Hydrochlorid

Eine Lösung von 2,25 g Boc-Phe-Gaba-OBzl (Stufe 1.1) in 2,2 ml 90 Vol.% wässrigem Trifluoräthanol wird mit 12,8 ml 1,2 N Salzsäure in 90 Vol.% Trifluoräthanol versetzt. Nach 30 Minuten werden 50 ml tert-Butanol zugefügt, die Lösung im Vakuum eingeengt und das restliche Lösungsmittel durch Lyophilisieren entfernt. Der Rückstand wird aus Aethylacetat umkristallisiert, Smp: 101-102°
DC: [Chloroform-Methanol (8:2)] Rf 0,49.

Stufe 1.3 Z-Phe-Phe-(D-Trp)-Lys(Boc)-Thr(But)-Phe-Phe-Gaba-OBzl

233 mg Z-Phe-Phe-(D-Trp)-Lys(Boc)-Thr(But)-Phe-OH (vergl. die genn. Eur. Anmeldung, Beispiel 7.2), 94 mg H-Phe-Gaba-OBzl-Hydrochlorid (Stufe 1.2) und 46 mg 1-Hydroxybenzotriazolmonohydrat werden in 0,5 ml DMF warm gelöst. Nach Abkühlen auf -10° werden 28 µl N-Methylmorpholin und eine Lösung von 53 mg DCCI in 0,1 ml DMF zugegeben, noch 1 Stunde gerührt und 20 Stunden bei -5° belassen. Die dicke Masse wird mit Aether zerrieben, das Ungelöste abgenutscht und noch mit kaltem Methanol gewaschen. Ausser Spuren Dicyclohexylharnstoff ist das Produkt laut DC rein.
DC: [Chloroform-Methanol (95:5)] Rf 0,42.

Stufe 1.4 H-Phe-Phe-(D-Trp)-Lys(Boc)-Thr(But)-Phe-Phe-Gaba-OH

Eine Lösung von 250 mg Z-Phe-Phe-(D-Trp)-Lys(Boc)-Thr(But)-Phe-Phe-Gaba-OBzl (Stufe 1.3) in 15 ml DMF wird nach Zugabe von 25 mg Palladium-Kohle (10 %) während 2 Stunden hydriert. Nach Abfiltrieren des Katalysators wird das Filtrat im Hochvakuum zur Trockne eingedampft, und der Rückstand ohne weitere Reinigung für die Cyclisierung verwendet.
DC: [System 157A] Rf 0,12.

Beispiel 2: [D-Trp$^{8}$, D-Phe$^{12}$, Gaba$^{13}$]-cyclo-somatostatin(6-13)-octapeptid.

cyclo[Phe-Phe-(D-Trp)-Lys-Thr-Phe-(D-Phe)-Gaba]

In analoger Weise wie im Beispiel 1 beschrieben, cyclisiert man H-Phe-Phe-(D-Trp)-Lys(Boc)-Thr(But)-Phe-(D-Phe)-Gaba-OH mittels DCCI und 1-Hydroxybenzotriazol. Das Rohprodukt wird durch Gegenstromverteilung gereinigt. (K = 0,3)
DC : [System 157A] Rf 0,49

Das erhaltene cyclische Peptid der Formel

cyclo[Phe-Phe-(D-Trp)-Lys(Boc)-Thr(But)-Phe-(D-Phe)-Gaba]

wird analog Beispiel 1 mit Trifluoressigsäure behandelt und durch Gegenstromverteilung gereinigt (K=0,67).
DC: System 157C : Rf 0,32
100 : Rf 0,32

Das Ausgangsmaterial wird wie folgt erhalten:

Stufe 2.1 Boc-(D-Phe)-Gaba-OBzl

Die Verbindung wird aus Boc-(D-Phe)-OH und H-Gaba-OBzl-p-Toluolsulfonat in analoger Weise hergestellt, wie im Beispiel 1, Stufe 1.1 beschrieben.
DC: [Toluol-Aethylacetat(1:9)] Rf 0,54.

Stufe 2.2 H-(D-Phe)-Gaba-OBzl-Hydrochlorid

Diese Verbindung wird in analoger Weise wie im Beispiel 1, Stufe 1.2, durch Abspaltung der Boc-Gruppen aus Boc-(D-Phe)-Gaba-OBzl erhalten. Smp: 99-100°
DC: [Chloroform-Methanol (8:2)] Rf 0,49.

Stufe 2.3 Z-Phe-Phe-(D-Trp)-Lys(Boc)-Thr(But)-Phe-(D-Phe)-Gaba-OBzl

Die Verbindung wird in analoger Weise wie im Beispiel 1, Stufe 1.3, durch Kondensation von Z-Phe-Phe-(D-Trp)-Lys(Boc)-Thr(But)-Phe-OH und H-(D-Phe)-Gaba-OBzl-Hydrochlorid (Stufe 2.2) mit DCCI in Gegenwart von 1-Hydroxybenzotriazol und N-Methylmorpholin erhalten.
DC: [System 157A Rf 0,68.

Stufe 2.4. H-Phe-Phe-(D-Trp)-Lys(Boc)-Thr(But)-Phe-(D-Phe)-Gaba-OH

Diese Verbindung wird in analoger Weise wie im Beispiel 1, Stufe 1.4, durch Hydrierung von Z-Phe-Phe-(D-Trp)-Lys(Boc)-Thr(But)-Phe-(D-Phe)-Gaba-OBzl (Stufe 2.3) erhalten und ohne Reinigung cyclisiert.

Patentansprüche (für alle benannten Länder ausser Oesterreich)

1. Verfahren zur Herstellung eines cyclischen Octapeptids der Formel

$$\overline{\text{Phe-Phe-trp-Lys}(Ac_A)\text{-Thr-Phe-A-B}} \quad (I),$$

6 7 8 9 10 11 12 13

worin trp für L-Trp, D-Trp oder einen analogen Rest, der im Indolkern ein Halogen trägt, $Ac_A$ für Wasserstoff oder einen an der ε-Aminogruppe befindlichen Acylrest Ac einer Carbonsäure, A für L-Phe, D-Phe, L-Phg oder D-Phg, oder einen entsprechenden Rest mit substituiertem oder gesättigtem Phenylring, und B für den Rest einer γ- oder δ-Aminoniederalkancarbonsäure, welche einen gegebenenfalls substituierten cyclischen Hydrocarbylrest Ar tragen kann, steht, sowie von Salzen und Komplexen davon, dadurch gekennzeichnet, dass man in einer strukturell entsprechenden cyclischen Verbindung, in welcher mindestens eine von vorhandenen Amino-, Hydroxyl- und/oder Carboxyl-Gruppen eine Aminoschutzgruppe X, eine Hydroxylschutzgruppe Y, bzw. eine Carboxylschutzgruppe W trägt, die Schutzgruppe(n) abspaltet und wenn erwünscht, in einer erhaltenen Verbindung mit freier ε-Aminogruppe des $Lys^9$-Restes an dieser Aminogruppe acyliert, und/oder, wenn erwünscht, eine erhaltene Verbindung basischen Charakters in ein Säureadditionssalz davon, oder ein solches Säureadditionssalz in die entsprechende Base umwandelt, oder, wenn erwünscht, eine erhaltene Verbindung sauren Charakters in ein Salz davon, oder ein solches Salz in die entsprechende saure Verbindung umwandelt, und/oder, wenn erwünscht, eine erhaltene Verbindung in ihr Komplex überführt.

2. Verfahren gemäss Anspruch 1 zur Herstellung eines cyclischen Octapeptids der Formel

$$\overline{\text{Phe-Phe-trp-Lys-Thr-Phe-A-B}} \quad (IA),$$

worin trp für L-Trp, D-Trp oder einen analogen Rest, der im Indolkern ein Halogen trägt, A für L-Phe, D-Phe, L-Phg oder D-Phg, oder einen entsprechenden Rest mit substituiertem oder gesättigtem Phenylring, und B für den Rest einer γ- oder δ-Aminoniederalkancarbonsäure, welche einen gegebenenfalls substituierten cyclischen Hydrocarbylrest Ar tragen kann, steht, sowie von Salzen und Komplexen davon, dadurch gekennzeichnet, dass man in einem entsprechenden Peptid der Formel

$$\lfloor\text{Phe-Phe-trp-Lys}(X_o)\text{-Thr}(Y_o)\text{-A-B}\rfloor \qquad \text{(III)} \quad ,$$

worin Thr, A und B die oben genannten Bedeutungen haben, $X_o$ eine ε-Aminoschutzgruppe X oder Wasserstoff und $Y_o$ eine Hydroxylschutzgruppe Y oder Wasserstoff bedeutet, wobei nur eines der Symbole $X_o$ und $Y_o$ Wasserstoff bedeuten kann, die Schutzgruppe(n) abspaltet und, wenn erwünscht, eine erhaltene Verbindung basischen Charakters in ein Säureadditionssalz davon, oder ein erhaltenes Säureadditionssalz in die entsprechende Base umwandelt, und/oder, wenn erwünscht, eine erhaltene Verbindung in ihr Komplex überführt.

3. Verfahren gemäss Anspruch 1 oder 2, dadurch gekennzeichnet, dass man ein lineares Peptid der Formel

$$\text{H-}[I_a]\text{-V} \qquad \text{(II)} \quad ,$$

worin $I_a$ einen der Formel I bzw. IA entsprechenden Rest darstellt, in welchem die amidische Bindung zwischen zwei beliebigen benachbarten Aminosäureresten des Peptidringes unterbrochen ist, und V für eine freie Hydroxylgruppe, eine durch eine Aktivierungsgruppe abgewandelte Hydroxylgruppe oder die Hydrazinogruppe $-NH-NH_2$ steht, cyclisiert, wobei übrige vorhandene, an der Cyclisierungsreaktion nicht beteiligte Amino-, Carboxyl- und Hydroxylgruppen nach Bedarf in geschützter Form vorliegen und anschliessend freigesetzt werden, und

wenn erwünscht, in einer erhaltenen Verbindung mit freier ε-Aminogruppe des $Lys^9$-Restes an dieser Aminogruppe acyliert, und/oder, wenn erwünscht, eine erhaltene Verbindung basischen Charakters in ein Säureadditionssalz davon, oder ein solches Säureadditionssalz in die entsprechende Base umwandelt, oder, wenn erwünscht, eine erhaltene Verbindung sauren Charakters in ein Salz davon, oder ein solches Salz in die entsprechende saure Verbindung umwandelt, und/oder, wenn erwünscht, eine erhaltene Verbindung in ihr Komplex überführt.

4. Ein cyclisches Octapeptid der Formel

$$\overline{\text{Phe-Phe-trp-Lys}(Ac_A)\text{-Thr-Phe-A-B}} \qquad (I),$$

6 7 8 9 10 11 12 13

worin trp für L-Trp, D-Trp oder einen analogen Rest, der im Indolkern ein Halogen trägt, $Ac_A$ für Wasserstoff oder einen an der ε-Aminogruppe befindlichen Acylrest Ac einer Carbonsäure, A für L-Phe, D-Phe, L-Phg oder D-Phg, oder einen entsprechenden Rest mit substituiertem oder gesättigtem Phenylring, und B für den Rest einer γ- oder δ-Aminoniederalkancarbonsäure, welche einen gegebenenfalls substituierten cyclischen Hydrocarbylrest Ar tragen kann, steht, sowie pharmakologisch verträgliche Salze und therapeutisch verwendbare Komplexe davon.

5. Ein cyclisches Octapeptid gemäss Anspruch 4, worin in der Formel I trp für D-Trp oder D-(5F)Trp steht, $Ac_A$ Wasserstoff oder den Acylrest Ac bedeutet, A für L-Phe, D-Phe, L-Phg oder D-Phg, oder einen entsprechenden im Phenylring gesättigten Rest, und B für den Rest einer γ- oder δ-Aminoniederalkancarbonsäure mit 4 bis 8 C-Atomen steht, sowie pharmakologisch verträgliche Salze und therapeutisch verwendbare Komplexe davon.

6. Ein cyclisches Peptid gemäss Anspruch 4, worin in der Formel I trp für D-Trp oder D-(5F)Trp steht, $Ac_A$ Wasserstoff bedeutet, A für

L-Phe, D-Phe, L-Phg oder D-Phg, oder einen entsprechenden im Phenylring gesättigten Rest, und B für den Rest der γ-Aminobuttersäure oder der δ-Aminovaleriansäure steht, sowie pharmakologisch verträgliche Salze und therapeutisch verwendbare Komplexe davon.

7. [D-$Trp^{8}$,$Phe^{12}$,$Gaba^{13}$]-cyclo-somatostatin(6-13)octapeptid, sowie pharmakologisch verträgliche Salze und therapeutisch verwendbare Komplexe davon.

8. [D-$Trp^{8}$,D-$Phe^{12}$,$Gaba^{13}$]-cyclo-somatostatin(6-13)-octapeptid, sowie pharmakologisch verträgliche Salze und therapeutisch verwendbare Komplexe davon.

9. Eine Verbindung gemäss einem der Ansprüche 4 bis 8 als Antidiabeticum.

10. Ein pharmazeutisches Präparat enthaltend mindestens eine Verbindung gemäss einem der Ansprüche 4 bis 9.

Patentansprüche (für Oesterreich)

1. Verfahren zur Herstellung eines cyclischen Octapeptids der Formel

$$\overline{\text{Phe-Phe-trp-Lys}(Ac_A)\text{-Thr-Phe-A-B}}$$ (I) ,

6 7 8 9 10 11 12 13

worin trp für L-Trp, D-Trp oder einen analogen Rest, der im Indolkern ein Halogen trägt, $Ac_A$ für Wasserstoff oder einen an der $\varepsilon$-Aminogruppe befindlichen Acylrest Ac einer Carbonsäure, A für L-Phe, D-Phe, L-Phg oder D-Phg, oder einen entsprechenden Rest mit substituiertem oder gesättigtem Phenylring, und B für den Rest einer $\gamma$- oder $\delta$-Aminoniederalkancarbonsäure, welche einen gegebenenfalls substituierten cyclischen Hydrocarbylrest Ar tragen kann, steht, sowie von Salzen und Komplexen davon, dadurch gekennzeichnet, dass man in einer strukturell entsprechenden cyclischen Verbindung, in welcher mindestens eine von vorhandenen Amino-, Hydroxyl- und/oder Carboxyl-Gruppen eine Aminoschutzgruppe X, eine Hydroxylschutzgruppe Y, bzw. eine Carboxylschutzgruppe W trägt, die Schutzgruppe(n) abspaltet und wenn erwünscht, in einer erhaltenen Verbindung mit freier $\varepsilon$-Aminogruppe des $Lys^9$-Restes an dieser Aminogruppe acyliert, und/oder, wenn erwünscht, eine erhaltene Verbindung basischen Charakters in ein Säureadditionssalz davon, oder ein solches Säureadditionssalz in die entsprechende Base umwandelt, oder, wenn erwünscht, eine erhaltene Verbindung sauren Charakters in ein Salz davon, oder ein solches Salz in die entsprechende saure Verbindung umwandelt, und/oder, wenn erwünscht, eine erhaltene Verbindung in ihr Komplex überführt.

2. Verfahren gemäss Anspruch 1 zur Herstellung eines cyclischen Octapeptids der Formel

$$\overline{\text{Phe-Phe-trp-Lys-Thr-Phe-A-B}}$$ (IA) ,

BAD ORIGINAL

worin trp für L-Trp, D-Trp oder einen analogen Rest, der im Indolkern ein Halogen trägt, A für L-Phe, D-Phe, L-Phg oder D-Phg, oder einen entsprechenden Rest mit substituiertem oder gesättigtem Phenylring, und B für den Rest einer γ- oder δ-Aminoniederalkancarbonsäure, welche einen gegebenenfalls substituierten cyclischen Hydrocarbylrest Ar tragen kann, steht, sowie von Salzen und Komplexen davon, dadurch gekennzeichnet, dass man in einem entsprechenden Peptid der Formel

$$\lfloor \text{Phe-Phe-trp-Lys}(X_o)\text{-Thr}(Y_o)\text{-A-B} \rfloor \quad \text{(III)} ,$$

worin Thr, A und B die oben genannten Bedeutungen haben, $X_o$ eine ε-Aminoschutzgruppe X oder Wasserstoff und $Y_o$ eine Hydroxylschutzgruppe Y oder Wasserstoff bedeutet, wobei nur eines der Symbole $X_o$ und $Y_o$ Wasserstoff bedeuten kann, die Schutzgruppe(n) abspaltet und, wenn erwünscht, eine erhaltene Verbindung basischen Charakters in ein Säureadditionssalz davon, oder ein erhaltenes Säureadditionssalz in die entsprechende Base umwandelt, und/oder, wenn erwünscht, eine erhaltene Verbindung in ihr Komplex überführt.

3. Verfahren gemäss Anspruch 1 oder 2, dadurch gekennzeichnet, dass man ein lineares Peptid der Formel

$$\text{H-}[I_a]\text{-V} \quad \text{(II)} ,$$

worin $I_a$ einen der Formel I bzw. IA entsprechenden Rest darstellt, in welchem die amidische Bindung zwischen zwei beliebigen benachbarten Aminosäureresten des Peptidringes unterbrochen ist, und V für eine freie Hydroxylgruppe, eine durch eine Aktivierungsgruppe abgewandelte Hydroxylgruppe oder die Hydrazinogruppe $-NH-NH_2$ steht, cyclisiert, wobei übrige vorhandene, an der Cyclisierungsreaktion nicht beteiligte Amino-, Carboxyl und Hydroxylgruppen nach Bedarf in geschützter Form vorliegen und anschliessend freigesetzt werden, und

wenn erwünscht, in einer erhaltenen Verbindung mit freier ε-Aminogruppe des $Lys^9$-Restes an dieser Aminogruppe acyliert, und/oder, wenn erwünscht, eine erhaltene Verbindung basischen Charakters in ein Säureadditionssalz davon, oder ein solches Säureadditionssalz in die entsprechende Base umwandelt, oder, wenn erwünscht, eine erhaltene Verbindung sauren Charakters in ein Salz davon, oder ein solches Salz in die entsprechende saure Verbindung umwandelt, und/oder, wenn erwünscht, eine erhaltene Verbindung in ihr Komplex überführt.

4. Verfahren gemäss einem der Ansprüche 1 bis 3, dadurch gekennzeichnet, dass man ein cyclisches Octapeptid der Formel I herstellt, worin trp für D-Trp oder D-(5F)Trp steht, $Ac_A$ Wasserstoff oder den Acylrest Ac bedeutet, A für L-Phe, D-Phe, L-Phg oder D-Phg, oder einen entsprechenden im Phenylring gesättigten Rest, und B für den Rest einer γ- oder ε-Aminoniederalkancarbonsäure mit 4 bis 8 C-Atomen steht, oder ein pharmakologisch verträgliches Salz, oder einen therapeutisch verwendbaren Komplex davon.

5. Verfahren gemäss einem der Ansprüche 1 bis 3, dadurch gekennzeichnet, dass man ein cyclisches Peptid der Formel I herstellt, worin trp für D-Trp oder D-(5F)Trp steht, $Ac_A$ Wasserstoff bedeutet, A für L-Phe, D-Phe, L-Phg oder D-Phg, oder einen entsprechenden im Phenylring gesättigten Rest, und B für den Rest der γ-Aminobuttersäure oder der δ-Aminovaleriansäure steht, oder ein pharmakoligisch verträgliches Salz oder einen therapeutisch verwendbaren Komplex davon.

6. Verfahren gemäss einem der Ansprüche 1 bis 3, dadurch gekennzeichnet, dass man [$D\text{-}Trp^8$,$Phe^{12}$,$Gaba^{13}$]-cyclo-somatostatin(6-13)-octapeptid oder ein pharmakologisch verträgliches Salz oder einen therapeutisch verwendbaren Komplex davon herstellt.

BAD ORIGINAL

7. Verfahren gemäss einem der Ansprüche 1 bis 3, dadurch gekennzeichnet, dass man [D-Trp$^{8}$,D-Phe$^{12}$,Gaba$^{13}$]-cyclo-somatostatin(6-13)-octapeptid oder ein pharmakologisch verträgliches Salz oder einen therapeutisch verwendbaren Komplex davon herstellt.

8. Verfahren zur Herstellung eines pharmazeutischen Präparates auf nicht-chemischem Wege, welches Präparat eine gemäss einem der Ansprüche 1 bis 7 hergestellte Verbindung enthält.

9. Verfahren gemäss Anspruch 8, dadurch gekennzeichnet, dass man ein Arzneimittel zur Verwendung als Antidiabeticum herstellt.

10. Verfahren zur Herstellung eines linearen Peptids der Formel

$$H-[I_a]-V \qquad (II),$$

worin $I_a$ einen der im Anspruch 1 definierten Formel I entsprechenden Rest darstellt, in welchem die amidische Bindung zwischen zwei beliebigen benachbarten Aminosäure-Resten des Peptidringes unterbrochen ist, und V für eine freie Hydroxylgruppe, eine durch eine Aktivierungsgruppe abgewandelte Hydroxylgruppe oder die Hydrazinogruppe $-NH-NH_2$ steht, dadurch gekennzeichnet, dass man die zu dessen Aufbau nötigen Aminosäuren bzw. kleinere Peptideinheiten unter Bildung von CONH-Bindungen in beliebiger zeitlicher Reihenfolge miteinander kondensiert, wobei nicht an der Reaktion teilnehmende funktionelle Gruppen intermediär geschützt werden können, und wenn erwünscht, eine erhaltene Verbindung mit freier Carboxylgruppe in einen entsprechenden Aktivester bzw. Hydrazid umwandelt.

BAD ORIGINAL